# EUROPEAN PATENT SPECIFICATION

(11) **EP 3 952 787 B1**
(45) Date of publication and mention of the grant of the patent: **11.03.2026**
(21) Application number: 20788061.8
(22) Date of filing: 08.04.2020
(51) Int. Cl.: A61F 2/06, A61F 2/82, A61F 2/95, A61F 2/86

(54) **PATENT DUCTUS ARTERIOSUS STENT**
DUCTUS-ARTERIOSUS-STENT FÜR PATIENTEN
ENDOPROTHÈSE DE CANAL ARTÉRIEL DE PATIENT

(30) Priority: 08.04.2019 US 201962830641 P
(43) Date of publication of application: 16.02.2022
(73) Proprietor: Vanderbilt University, Nashville, TN 37240 (US)
(72) Inventor: DOYLE, Thomas, Nashville, Tennessee 37232 (US); JANSSEN, Dana, Nashville, Tennessee 37232 (US); NICHOLSON, George Thomas, Nashville, Tennessee 37232 (US)
(74) Representative: Papula Oy
(86) International application number: PCT/US2020/027187
(87) International publication number: WO 2020/210299

(56) References cited:
- US-A- 6 004 316
- US-A1- 2002 099 437
- US-A1- 2006 259 119
- US-A1- 2011 106 234
- US-A1- 2011 295 353
- US-A1- 2015 081 000
- US-A1- 2018 318 114

## Description

### Related Application

This application claims the benefit of U.S. Provisional Patent Application Serial No. 62/830,641, filed April 8, 2019, entitled "PATENT DUCTUS STENT".

### Technical Field

The present disclosure relates to a stent and method for stenting the ductus arteriosus in order to keep the ductus arteriosus open. More particularly, the present disclosure relates to a stent configured to be positioned across the ductus arteriosus in a pediatric human patient in order to keep the ductus arteriosus open and ensure adequate blood flow.

### Background of the Invention

Patent ductus arteriosus (PDA) is a congenital disorder in the heart wherein a neonate's ductus arteriosus fails to close after birth. In a developing fetus, the ductus arteriosus is the vascular connection between the pulmonary artery and the aortic arch that allows most of the blood flow from the right ventricle to bypass the fetus' fluid-filled compressed lungs. During fetal development, this shunt protects the right ventricle from pumping against the high resistance in the lungs, which can lead to right ventricular failure if the ductus arteriosus closes in utero.

When a newborn takes his or her first breath, the lungs open and the pulmonary vascular resistance decreases. After birth, the placenta is separated from the infant reducing prostaglandin which keeps the ductus arteriosus open in-utero. In addition, the lungs release bradykinin to constrict the smooth muscle wall of the ductus arteriosus and reduce blood flow through the ductus arteriosus as it narrows and completely closes, usually within the first few weeks of life. If the patent ductus arteriosus does not close, long-term complications can arise, such as pulmonary hypertension, heart failure, and bacterial endocarditis.

In certain forms of congenital heart disease it is necessary to keep the ductus arteriosus open. In the short term this can be accomplished with IV prostaglandin. However, for long term patency, cardiologists have relied on vascular stents. These stents require a large stent to blood vessel surface area ratio in order to provide support, as these stents are not specifically designed to keep a ductus arteriosus patent. When the vascular stents are implanted in the PDA, they become incorporated into the vascular tissue. Once incorporated, the vascular stents can be difficult to remove, resulting in trauma to the vessels and surrounding structures. Thus, a stent specifically designed to keep the ductus arteriosus open, and that can be removed in an atraumatic manner, is desirable.

US2018318114A1 discloses a retrievable implant designed for placement in a prostatic urethra narrowed by enlarged tissue. It features a proximal hub and a distal hub connected by a plurality of arms. In its expanded configuration, the arms deploy outward in a predetermined shape to retract the enlarged tissue, thereby increasing the urethral lumen. The implant is designed to be atraumatic and is retrievable, allowing for minimally invasive procedures. However, it does not suggest a stent configured to fit the natural curved shape of the ductus arteriosus, nor does it address the specific curvature of struts that curve toward and away from the longitudinal axis, as required to maintain the natural shape of the ductus arteriosus and allow for easy retrieval.

### Summary of the Disclosure

In one aspect of the present disclosure, a stent is provided according to claim 1. When the stent is positioned in the ductus arteriosus, the curved proximal strut ends and the curved distal strut ends engage the ductus arteriosus to provide the radial force necessary to keep the ductus arteriosus open while the elongated strut body portions of each of the 4 to 8 struts minimally engage the ductus arteriosus to prevent invagination into the vascular wall of the ductus arteriosus and thus allow for subsequent retrieval.

In another aspect of the present disclosure, a method of stenting the ductus arteriosus in a pediatric human patient in order to ensure adequate blood flow is provided. One step of the method can include providing a retrievable stent. The retrievable stent can include a proximal end connector and a distal end tip disposed about a longitudinal axis. The proximal end connector can include one or more retrieval mechanisms and a proximal end connector lumen that runs the length of the proximal end connector. The distal end tip can have a distal end tip lumen that runs the length of the distal end tip. The retrievable strut can also include a plurality of 4 to 8 struts extending from the proximal end connector to the distal end tip, each of the struts having a curved proximal strut end and a curved distal strut end where the curved proximal strut end and the curved distal strut end extend radially outward from the longitudinal axis and toward one another. Each of the struts can have an elongated strut body portion that extends from the curved proximal strut end to the curved distal strut end. The elongated strut body portions can extend parallel to each other. Another step of the method can include positioning the retrievable stent in the ductus arteriosus of the pediatric human patient so that the stent struts are parallel with the vascular wall of the ductus arteriosus and so that the curved proximal strut ends and the curved distal strut ends engage the ductus arteriosus to provide the radial force necessary to keep the ductus arteriosus open while the elongated strut body portions of each of the 4 to 8 struts minimally engage the ductus arteriosus to prevent invagination into the vascular wall of the ductus arteriosus.

### Brief Description of the Drawings

The foregoing and other features of the present disclosure will become apparent to those skilled in the art to which the present disclosure relates upon reading the following description with reference to the accompanying drawings, in which:
Fig. 1 is a perspective view showing an exemplary retrievable stent for keeping the ductus arteriosus open in accordance with one aspect of the present disclosure;
Figs. 2A-B show a magnified view of (A) a hook retrieval mechanism, and (B) a hook retrieval mechanism with one half of a threaded fastener retrieval mechanism in accordance with another aspect of the present disclosure;
Fig. 3 is a perspective view showing a second exemplary retrievable stent for keeping the ductus arteriosus open in accordance with a further aspect of the present disclosure;
Fig. 4 is a perspective view of the invention showing a third exemplary retrievable stent for keeping the ductus arteriosus open in accordance with another aspect of the present disclosure;
Figs. 5A-H are cross-sectional views showing exemplary radial distributions of the stent struts in accordance with other aspects of the present disclosure;
Fig. 6 shows a method of stenting the ductus arteriosus in a pediatric human patient in order to ensure adequate blood flow in accordance with one aspect of the present disclosure;
Fig. 7 is a schematic illustration showing an exemplary stent placed across the ductus arteriosus in accordance with another aspect of the present disclosure;
Fig. 8 is an image showing a guidewire being passed through an exemplary stent in accordance with one aspect of the present disclosure; and
Fig. 9 is an image showing an exemplary stent attached to a delivery cable in accordance with another aspect of the present invention.

### Detailed Description

The present disclosure relates generally to a stent configured to keep the ductus arteriosus open in a pediatric human patient. The ductus arteriosus is a unique blood vessel that typically closes naturally within 10 days after birth. In many forms of congenital heart disease, such as with cyanotic heart defects, it is critical to keep the ductus arteriosus open (i.e., patent) in order to ensure either adequate systemic or pulmonary blood flow, depending on the cardiac lesion. As used herein, the term "patent" is a medical term that refers to a structure that is generally open and/or unobstructed, and which is not substantially closed. Cyanotic heart defects are a type of congenital heart defect in which a patient appears blue (cyanotic) due to deoxygenated blood bypassing the lungs and entering the systemic circulation. Cyanotic heart defects include, but are not limited to, hypoplastic left heart syndrome (HLHS), Tetralogy of Fallot (ToF), total anomalous pulmonary venous connection, transposition of the great arteries (d-TGA), truncus arteriosus, tricuspid atresia, interrupted aortic arch, coarctation of aorta, pulmonary atresia (PA), and pulmonary stenosis. The patent ductus arteriosus provides a pathway to ensure either adequate systemic or pulmonary circulation, depending on the specific type of congenital heart disease. Keeping the ductus arteriosus open is necessary until a pediatric patient is able to grow and become a better candidate for surgical repair.

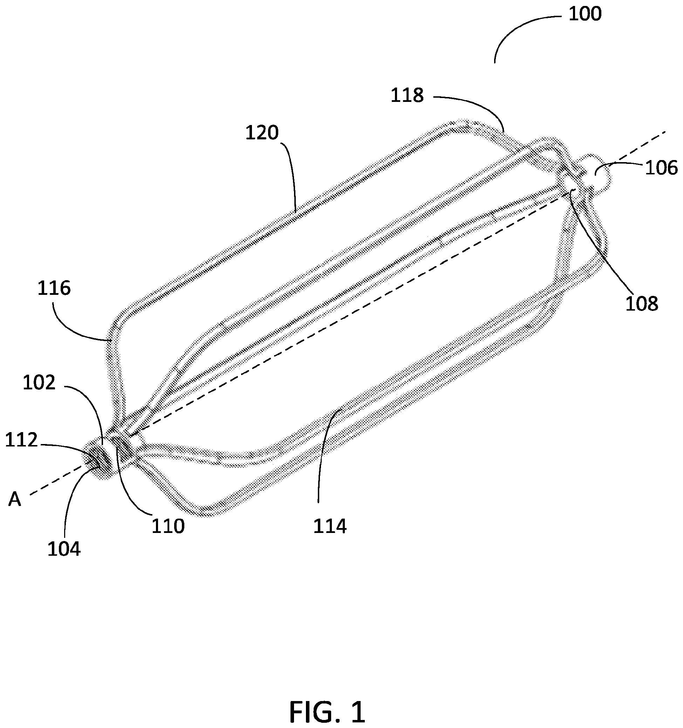

Current mechanical methods for keeping the ductus arteriosus open use a peripheral vascular stent or a coronary stent in an off-label fashion. These currently available stents are far from optimal for keeping the ductus arteriosus open because these stents need to be removed surgically and they cannot be left in place for an extended period of time. These currently available stents are also problematic because they have extensive metal crosslinking latticework that becomes densely incorporated into the blood vessel wall. As a result, surgical removal becomes difficult and hazardous, and can result in vascular injury and/or nerve damage. As representative of one aspect of the present disclosure, Fig. 1 illustrates a retrievable stent 100 configured to be positioned across the ductus arteriosus in a pediatric human patient to keep the ductus arteriosus open and to ensure adequate blood flow. As described in more detail below, aspects of the retrievable stent 100 described in the present disclosure allow for the ductus arteriosus to be kept open in a manner that allows for atraumatic removal of the retrievable stent following even lengthy use of the retrievable stent in a pediatric human patient.

Figs. 1, 2, 3, 4, and 5 illustrate a retrievable stent 100 for use in keeping the ductus arteriosus open in a pediatric human patient. As used herein, the term "pediatric human patient" refers to a human under 12 months of age. As shown in Fig. 1, the retrievable stent 100 can include a proximal end connector 102 and a proximal end connector lumen 104 that runs the length of the proximal end connector 102. The retrievable stent 100 can also include a distal end tip 106 and a distal end tip lumen 108 that runs the length of the distal end tip 106. In one aspect, the proximal end connector lumen 104 and the distal end tip lumen 108 each have a diameter large enough such that a guidewire is able to run through their respective lumens.

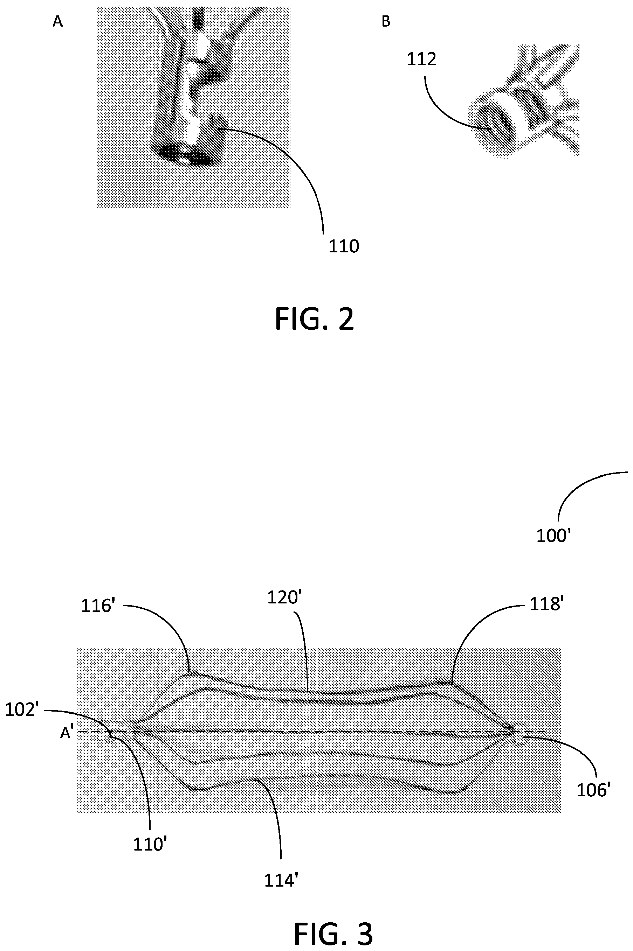

The proximal end connector 102 can include one or more retrieval mechanisms. As shown in Fig. 2, the retrieval mechanism can include *e.g.,* a hook 110 and/or one half of a threaded fastener 112. One skilled in the art would appreciate that a variety of hook designs can be used. For example, the hook may be rounded or have a ball shape or the hook may be rectangular or square in shape.

The retrievable stent 100 can include 4 to 8 struts 114 that can extend from the proximal end connector 102 to the distal end tip 106. In one instance, the retrievable stent 100 can have 4 struts. In another instance, the retrievable stent 100 can have 5 struts. In a further instance, the retrievable stent 100 can have 6 struts. In yet another instance, the retrievable stent 100 can have 7 struts. In another instance, the retrievable stent 100 can have 8 struts. Each of the struts 114 can have a curved proximal strut end 116 and a curved distal strut end 118, wherein the curved proximal strut end and the curved distal strut end extend radially outward from the longitudinal axis A and toward one another. In one instance, the curved proximal strut end 116 and the curved distal strut end 118 can have a sigmoidal curve shape. In some instances, the curved proximal strut end 116 and the curved distal strut end 118 can comprise a single sigmoidal curve. In other instances, the curved proximal strut end 116 and the curved distal strut end 118 can comprise a single arc.

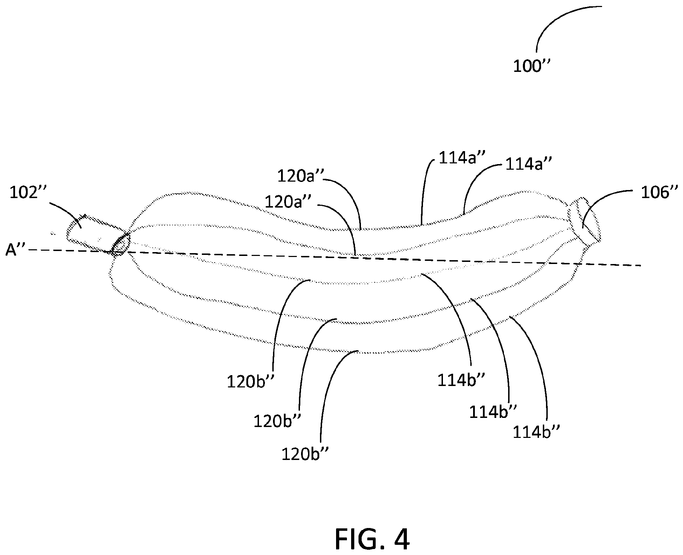

Each of the struts 114 can further comprise an elongated strut body portion 120 that extends from the curved proximal strut end 116 to the curved distal strut end 118. In one instance the elongated strut body portions 120 of each of the struts 114 can extend in a straight line. In certain instances when the elongated strut body portions 120 extend in a straight line, the elongated strut body portions can extend parallel to each other. Fig. 3 shows an alternative configuration of retrievable stent 100 designated as retrievable stent 100'. As seen in Fig. 3, the elongated strut body portions 120' of struts 114' of retrievable stent 100' can curve towards the longitudinal axis A'. Fig. 4 shows an example of the invention, by showing a configuration of retrievable stent 100 designated as retrievable stent 100" where struts 114" can vary in shape in such a manner that they cause retrievable stent 100" to have a banana shape. The retrievable stent 100" is designed to fit the natural curved shape of the ductus arteriosus and allow the ductus arteriosus to maintain its natural shape. In one instance, retrievable stent 100" can include one or more struts 114a" that have an elongated body portion 120a" that curves toward longitudinal axis A". Retrievable stent 100" can also include one or more struts 114b" that have an elongated body portion 120b" that curves away from longitudinal axis A".

The struts 114 can have any suitable cross-sectional shape. In some instances, the struts 114 can have a generally square cross-sectional shape. Other examples of suitable cross-sectional shapes include a generally circular cross-sectional shape, a generally rectangular cross sectional shape, a generally triangular cross-sectional shape, and a generally ovoid cross-sectional shape. In certain instances, the struts 114 can be *e.g.,* from 0.25 mm to 0.45 mm wide and from 0.2 mm to 0.35 mm thick. In one specific instance, the struts 114 can be 0.36 mm wide and 0.25 mm thick.

Experience has demonstrated that the entire length of the ductus arteriosus needs to be supported by the stent to prevent closure. Failure to adequately cover the length of the ductus arteriosus with the stent can allow natural constriction to occur, resulting in ductal closure despite the presence of the stent, and resultant death of the pediatric human patient. In one aspect, the retrievable stent 100 can have a length anywhere from 15 mm to 30 mm. In some instances, the length of retrievable stent 100 can be any length from 18 mm to 24 mm.

In one aspect, the diameter of retrievable stent 100 is large enough to span the vascular walls of the patent ductus arteriosus in the pediatric human patient. In some instances the diameter of retrievable stent 100 can be anywhere from 4 mm to 20 mm. In certain instances, the diameter of retrievable stent 100 can be from *e.g.,* 4 mm to 12 mm or from 6 mm to 10 mm. In one specific instance, the diameter of retrievable stent 100 can be 8 mm. In certain instances, the diameter of the retrievable stent 100 can vary along the longitudinal axis A such that the diameter of the retrievable stent 100 increases towards the distal end tip 106. In another instance, the diameter of the retrievable stent 100 can vary along the longitudinal axis A such that the diameter of the retrievable stent 100 increases towards the proximal end connector 102. In other instances in which struts 114' have an elongated strut body portion 120', the diameter of the retrievable stent 100 can vary along the longitudinal axis in accordance with the curve of the strut. In instances when the diameter of the retrievable stent varies along the longitudinal axis A, the diameter of retrievable stent 100 can vary anywhere from 4 mm to 20 mm. When retrievable stent 100 is positioned in the ductus arteriosus, the curved proximal strut ends 116 and the curved distal strut ends 118 can engage the ductus arteriosus to provide the radial force necessary to keep the ductus arteriosus open while the elongated strut body portions 120 of each of the struts 114 can minimally engage the ductus arteriosus to prevent endothelialization. In instances in which an elongated strut body portion 120' that curves towards the longitudinal axis A' is used, the elongated strut body portions only very minimally engage the vascular walls of the ductus arteriosus. One skilled in the art will appreciate that the length and diameter of the retrievable strut 100 used in a pediatric human patient can depend on the size of the pediatric human patient.

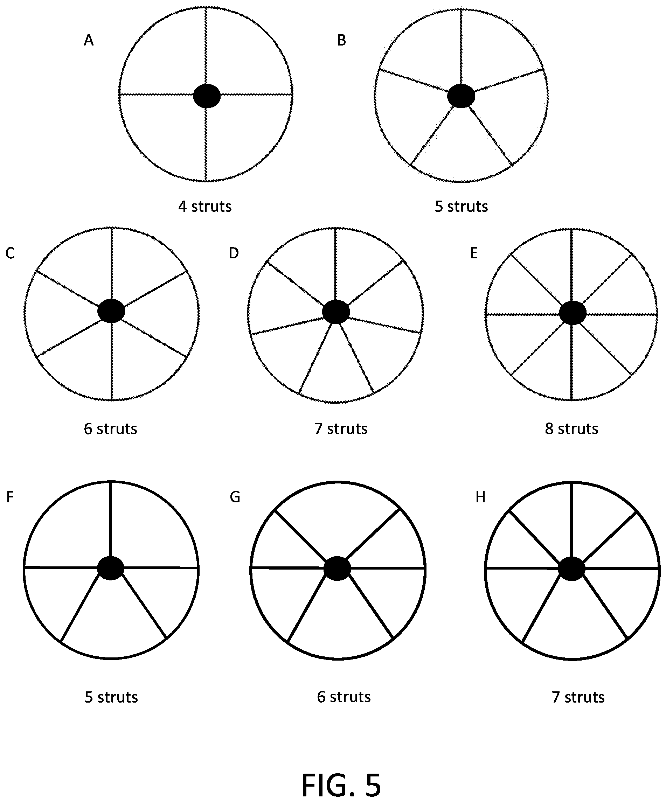

In another aspect, the plurality of 4 to 8 struts 114 used in retrievable stent 100 can be evenly spaced in a radial configuration. Fig. 5 illustrates various exemplary radial configurations of the struts 114 when 4, 5, 6, 7, and 8 struts are used. As seen in Figs. 5A-E, the struts 114 can be evenly spaced in a radial configuration. One skilled in the art would recognize that other configurations are possible including configurations where the struts 114 are not all evenly spaced. Exemplary configurations where the struts 114 are not all evenly spaced include those shown in Figs. 5F-H.

The proximal end connector lumen 104 and the distal end tip lumen 108 can each have a diameter large enough to allow a guide wire to run through their respective lumens. In certain instances, the diameter of the proximal end connector lumen 104 and the distal end tip lumen 108 can be between 4 mm to 16 mm. In certain instances, the diameter of the proximal end connector lumen 104 and the distal end tip lumen 108 are the same. In other instances, the diameter of the proximal end connector lumen 104 and the distal end tip lumen 108 are different.

The struts 114 can be formed of any suitable resilient material acceptable for use in implantable medical devices. Examples of suitable materials include, but are not limited to, stainless steel, nitinol, nickel-cobalt-chromium alloys, nickel-cobalt-chromium-molybdenum alloys, polymeric materials, and other biocompatible materials. Nickel-cobalt-chromium-molybdenum alloys, such as MP35N^{®}, can be used in some instances. In one instance, the struts 114 can comprise a shape memory material, such as nitnol. In one specific instance, the struts 114 are formed of nitinol.

The proximal end connector 102 and the distal end tip 106 can each be formed from the same material or different materials than that of the struts 114. In one instance, retrievable stent 100 comprises struts 114 made of nitinol and a proximal end connector 102 and a distal end tip 106 formed of stainless steel.

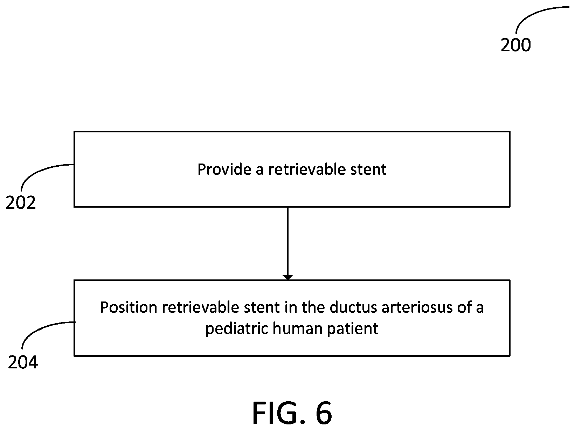

Figs. 6, 7, 8, and 9 illustrate another aspect of the present disclosure including methods of stenting the ductus arteriosus in a pediatric human patient in order to ensure adequate blood flow. For purposes of simplicity, the methods are shown and described as being executed serially; however, it is to be understood and appreciated that the present disclosure is not limited by the illustrated order as some steps could occur in different orders and/or concurrently with other steps shown and described herein.

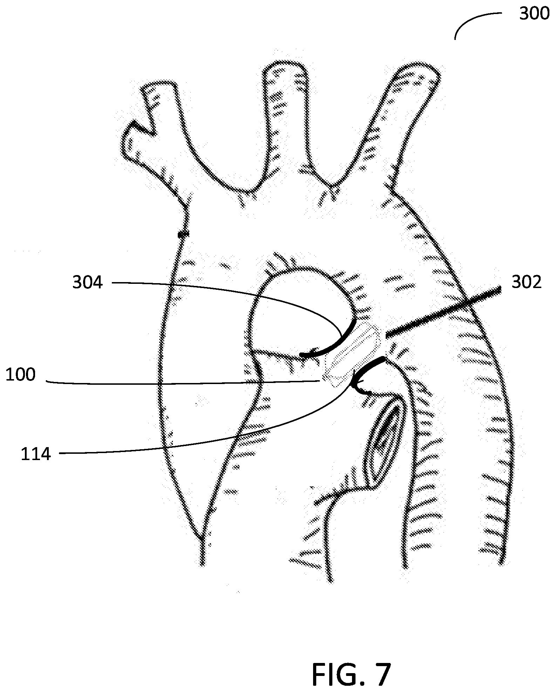

In one aspect, method 200 as shown in Fig. 6, provides an exemplary method of stenting the ductus arteriosus in a pediatric human patient in order to ensure adequate blood flow. At 202, a retrievable stent 100 can be provided. At step 204, the retrievable stent 100 can be positioned in the ductus arteriosus of a pediatric human patient. As shown in Fig. 7, the retrievable stent 100 can be positioned so that the stent struts 114 are parallel with the vascular walls 304 of the ductus arteriosus 302 in the heart 300.

In certain instances, a percutaneous method or other minimally invasive technique can be used to deliver the retrievable stent 100 to the ductus arteriosus 302. A guidewire can be inserted into a femoral access site, such as the femoral vein. Where the guidewire is inserted into a femoral access site, image guidance (e.g., fluoroscopy, ultrasound, magnetic resonance, computed tomography, or combinations thereof) can be used to direct the guidewire and catheter into and through the ductus arteriosus and into the descending aorta or pulmonary artery, ensuring stable guidewire position.

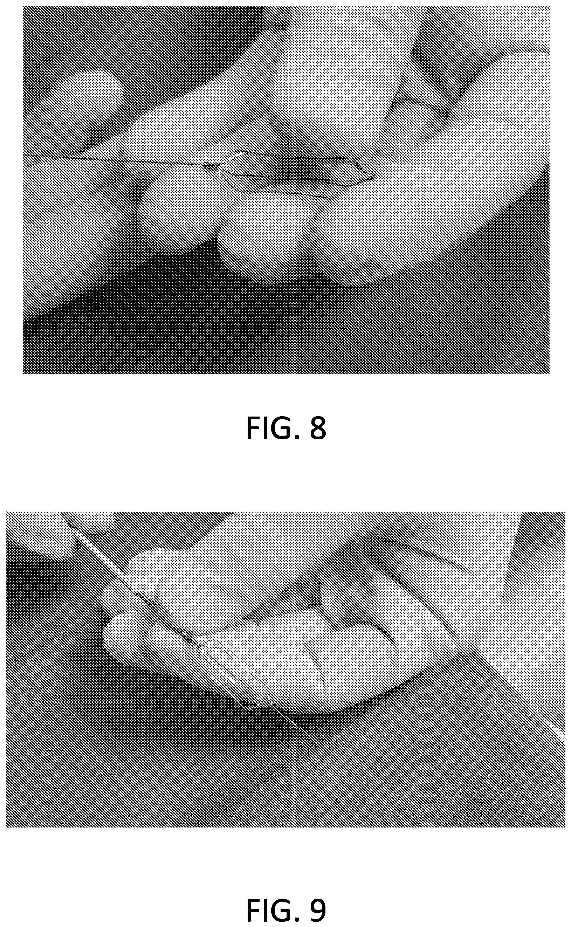

After the guidewire is secured in the pediatric human patient's heart, the guidewire can be passed through the proximal end connector lumen 104 and the distal end tip lumen 108 of retrievable stent 100 (Fig. 8). The retrievable stent 100 can then be attached to a delivery cable through the use of a threaded fastener (Fig. 9). The retrievable stent 100 can be pulled into the delivery cable causing the retrievable stent 100 to attain a compressed configuration. The delivery cable with compressed retrievable stent 100 can be passed over the guidewire and advanced to the ductus arteriosus 302.

Upon reaching the ductus arteriosus 302, the retrievable stent 100 can be freed from the delivery cable. Upon retraction of the delivery cable, the retrievable stent 100 can return to its original shape. As the retrievable stent 100 is freed from the delivery cable, the position of the retrievable stent 100 within the ductus arteriosus can be monitored, controlled, and/or quality assured by imaging systems of various kinds. For example, X-ray machines, fluoroscopic machines, ultrasound, CT, MRI, PET, and other imaging devices may be used.

Once the retrievable stent 100 is freed from the delivery cable, the retrievable stent 100 may be appropriately positioned in the ductus arteriosus 302. More particularly, the retrievable stent 100 can be positioned so that it covers the length of the ductus arteriosus as seen in Fig. 6. In one aspect, struts 114 of retrievable stent 100 can be positioned so that they are parallel with the vascular wall of the patent ductus arteriosus 302. In another aspect, the curved proximal strut ends 116 and the curved distal strut ends 118 of retrievable stent 100 can engage the ductus arteriosus to provide the radial force necessary to keep the ductus arteriosus open while the elongated strut body portions of each of the struts minimally engage the ductus arteriosus to prevent vascularization.

Once the retrievable stent 100 is positioned in the ductus arteriosus 302 of the pediatric human patient, the retrievable stent 100 can be disconnected from the delivery cable by disconnecting the threaded fastener. The delivery cable and guidewire can then be withdrawn from the pediatric human patient's vasculature. In certain instances in which the retrievable stent 100 is determined to be in a suboptimal position following disconnection of the delivery cable, the delivery cable can reengage and recapture the retrievable stent 100 either before or after the delivery cable has been removed from the pediatric human patient. Following reengagement and recapture of the retrievable stent 100, the retrievable stent 100 can then be repositioned in the ductus arteriosus. This process can be repeated as many times as necessary in order to achieve optimal placement of the retrievable stent 100.

Exemplary guidewires and delivery cables are known in the art. In one example, the delivery cable can be a catheter. The catheter can have a dimeter of *e.g.,* 5 or 6 French.

The retrievable stent 100 may remain in the ductus arteriosus of the pediatric human patient for up to 360 days. For example, the retrievable stent 100 may remain in the ductus arteriosus of the pediatric human patient for 1 to 360 days.

In one aspect, retrievable stent 100 can be removed from the pediatric human patient in an atraumatic manner. Atraumatic removal is facilitated by the features of retrievable stent 100, including *e.g*., the lack of extensive metal latticework, the parallel orientation of the struts 114 along the vascular wall, and the central location of the retrieval mechanism. The parallel orientation of the struts 114 along the vascular wall aids in atraumatic removal of retrievable stent 100 because the force required to remove any overgrown tissue is significantly less than that which would be required with a cross-linked stent structure. The single path of the struts 114 results in only one line of tissue being broken at any point, with minimal or no tissue being removed. In contrast, a cross-linked stent will tend to pull a patch of the inner tissue at each cross-link, resulting in significant damage to the vascular wall.

In certain instances, a cutting element may be needed to facilitate separation of the retrievable stent 100 from any overgrown tissue. Any known cutting elements can be used.

As shown in Fig. 1, a retrieval mechanism 110 can be located at the distal end of the retrievable stent 100 on the proximal end connector 102. The placement of the retrieval mechanism 110 allows the retrieval mechanism to be located centrally within the lumen of the blood vessel. This orientation provides for easy capture and retrieval of retrievable stent 100 and ensures that the retrieval mechanism 110 is not incorporated within the vascular wall due to vessel wall inflammation and somatic growth.

Any known retrieval devices can be used to remove the retrievable stent 100 from the pediatric human patient. For example, in instances where retrievable stent 100 includes a hook retrieval mechanism 110, a snare device can engage the hook retrieval mechanism and remove the retrievable 100 from the pediatric human patient. One skilled in the art would appreciate that any known snare devices can be used.

Unless otherwise defined, all technical terms used herein have the same meaning as commonly understood by one of ordinary skill in the art to which the present disclosure pertains.

In the context of the present disclosure, the singular forms "a," "an" and "the" can include the plural forms as well, unless the context clearly indicates otherwise. It will be further understood that the terms "comprises" and/or "comprising," as used herein, can specify the presence of stated features, steps, operations, elements, and/or components, but do not preclude the presence or addition of one or more other features, steps, operations, elements, components, and/or groups thereof.

As used herein, the term "and/or" can include any and all combinations of one or more of the associated listed items.

As used herein, phrases such as "between X and Y" and "from X to Y" can be interpreted to include X and Y. In the instance that X and Y are numerical values, "between X and Y" or "from X to Y" can be interpreted to include X and Y and all intervening values and ranges therein.

As used herein, when terms "distal" and "proximal" are used to refer to a portion of the stent, they refer to the device in its delivery configuration. The term "proximal" can refer to close to the operator (less into the body) and "distal" can refer to remote from the operator (further into the body).

From the above description of the invention, those skilled in the art will perceive improvements, changes and modifications. Such improvements, changes, and modifications are within the skill of the art and are intended to be covered by the appended claims.

## Claims

1. A retrievable stent (100") configured to be positioned across the ductus arteriosus in a pediatric human patient to keep the ductus arteriosus open and ensure adequate blood flow, wherein the retrievable stent (100") is configured to fit the natural curved shape of the ductus arteriosus and allow the ductus arteriosus to maintain its natural shape, the stent comprising:
a proximal end connector (102") and a distal end tip (106") disposed about a longitudinal axis (A"), the proximal end connector (102") including one or more retrieval mechanisms and a proximal end connector lumen (104") that runs the length of the proximal end connector (102"), and the distal end tip (106") having a distal end tip lumen (108") that runs the length of the distal end tip (106"), and
a plurality of 4 to 8 struts (114a", 114b") extending from the proximal end connector (102") to the distal end tip (106"), each of the struts having a curved proximal strut end (116") and a curved distal strut end (118") wherein the curved proximal strut end (116") and the curved distal strut end (118") extend radially outward from the longitudinal axis (A") and toward one another, each of the struts further having an elongated strut body portion (120a", 120b") that extends from the curved proximal strut end (116") to the curved distal strut end (118"), the elongated strut body portions (120a", 120b") extending parallel to each other;
wherein, when the stent (100") is positioned in the ductus arteriosus, the curved proximal strut ends (116") and the curved distal strut ends (118") engage the ductus arteriosus to provide the radial force necessary to keep the ductus arteriosus open while the elongated strut body (120a", 120b") portions of each of the 4 to 8 struts (114a", 114b") minimally engage the ductus arteriosus to prevent invagination into the vascular wall of the ductus arteriosus and thus allow for subsequent retrieval, and wherein one or more struts (114a") has an elongated body portion (120a") that curves toward the longitudinal axis (A") and one or more struts (114b") has an elongated body portion (120b") that curves away from longitudinal axis (A").

2. The stent of claim 1, wherein the curved proximal strut end (116") and the curved distal strut end (118") comprise a sigmoidal curve.

3. The stent of claim 1, wherein the retrieval mechanism comprises a hook (110") structure for receiving a snare.

4. The stent of claim 1, wherein the retrieval mechanism comprises a one half of a threaded fastener (112").

5. The stent of claim 1, comprising a hook (110") retrieval mechanism and one half of a threaded fastener (112") retrieval mechanism.

6. The stent of claim 1, wherein the lumen of the proximal end connector (104") and the lumen of the distal end tip (108") each have a diameter large enough such that a guidewire is able to run through their respective lumens (104", 108").

7. The stent of claim 1, comprising 5 struts (114").

8. The stent of claim 1, comprising 6 struts (114").

9. The stent of claim 1, comprising a length from 15 mm to 30 mm.

10. The stent of claim 1, comprising a length from 18 mm to 24 mm.

11. The stent of claim 1, comprising an outer diameter of from 4 mm to 20 mm.

12. The stent of claim 1, comprising an outer diameter of from 6 mm to 10 mm.

13. The stent of claim 1, wherein the struts (114") are formed of nitinol.

## Patentansprüche

1. Rückholbarer Stent (100") der dazu konfiguriert ist, entlang des Ductus arteriosus in einem pädiatrischen menschlichen Patienten positioniert zu werden, um den Ductus arteriosus offen zu halten und einen ausreichenden Blutfluss sicherzustellen, wobei der rückholbare Stent (100") dazu konfiguriert ist, sich der natürlichen gekrümmten Form des Ductus arteriosus anzupassen und es dem Ductus arteriosus zu erlauben, seine natürliche Form beizubehalten, wobei der Stent umfasst:
einen proximalen Endverbinder (102") und eine distale Endspitze (106"), die um eine longitudinale Achse (A") angeordnet sind, wobei der proximale Endverbinder (102") einen oder mehrere Rückholmechanismen und ein proximales Endverbinder-Lumen (104") enthält, das sich über die Länge des proximalen Endverbinders (102") erstreckt, und wobei die distale Endspitze (106") ein distales Endspitzenlumen (108") aufweist, das sich über die Länge der distalen Endspitze (106") erstreckt, und
eine Mehrzahl von 4 bis 8 Streben (114a", 114b"), die sich vom proximalen Endverbinder (102") zur distalen Endspitze (106") erstrecken, wobei jede der Streben ein gekrümmtes proximales Strebenende (116") und ein gekrümmtes distales Strebenende aufweist (118"), wobei sich das gekrümmte proximale Strebenende (116") und das gekrümmte distale Strebenende (118") radial nach außen von der longitudinalen Achse (A") und in Richtung zueinander erstrecken, wobei jede der Streben ferner einen länglichen Strebenkörperbereich (120a", 120b") aufweist, der sich vom gekrümmten proximalen Strebenende (116") zum gekrümmten distalen Strebenende (118") erstreckt, wobei die länglichen Strebenkörperbereiche (120a", 120b") sich parallel zueinander erstrecken;
wobei, wenn der Stent (100") im Ductus arteriosus positioniert ist, die gekrümmten proximalen Strebenenden (116") und die gekrümmten distalen Strebenenden (118") am Ductus arteriosus angreifen, um die radiale Kraft bereitzustellen, die erforderlich ist, um den Ductus arteriosus offen zu halten, während die länglichen Strebenkörperbereiche (120a", 120b") jeder der 4 bis 8 Streben (114a", 114b") minimal am Ductus arteriosus angreifen, um ein Einwachsen in die vaskuläre Wand des Ductus arteriosus zu verhindern und somit nachfolgend die Zurückholung zu erlauben, und wobei eine oder mehrere Streben (114a") einen länglichen Körperbereich (120a") aufweisen, der sich zur longitudinalen Achse (A") hin krümmt, und eine oder mehrere Streben (114b") einen länglichen Körperbereich (120b") aufweisen, der sich von der longitudinalen Achse (A") weg krümmt.

2. Stent nach Anspruch 1, wobei das gekrümmte proximale Strebenende (116") und das gekrümmte distale Strebenende (118") eine sigmoidale Krümmung umfassen.

3. Stent nach Anspruch 1, wobei der Rückholmechanismus eine Hakenstruktur (110") zum Aufnehmen einer Schlinge umfasst.

4. Stent nach Anspruch 1, wobei der Rückholmechanismus eine Hälfte eines Gewindebefestigungsmittels (112") umfasst.

5. Stent nach Anspruch 1, der einen Haken (110") als Rückholmechanismus und eine Hälfte eines Gewindebefestigungsmittels (112") als Rückholmechanismus umfasst.

6. Stent nach Anspruch 1, wobei das Lumen des proximalen Endverbinders (104") und das Lumen der distalen Endspitze (108") jeweils einen Durchmesser aufweisen, der groß genug ist, dass ein Führungsdraht durch ihre jeweiligen Lumina (104", 108") geführt werden kann.

7. Stent nach Anspruch 1, der 5 Streben (114") umfasst.

8. Stent nach Anspruch 1, der 6 Streben (114") umfasst.

9. Stent nach Anspruch 1, der eine Länge von 15 mm bis 30 mm umfasst.

10. Stent nach Anspruch 1, der eine Länge von 18 mm bis 24 mm umfasst.

11. Stent nach Anspruch 1, der einen Außendurchmesser von 4 mm bis 20 mm umfasst.

12. Stent nach Anspruch 1, der einen Außendurchmesser von 6 mm bis 10 mm umfasst.

13. Stent nach Anspruch 1, wobei die Streben (114") aus Nitinol gebildet sind.

## Revendications

1. Endoprothèse récupérable (100") configurée pour être positionnée à travers le canal artériel chez un patient humain pédiatrique pour maintenir le canal artériel ouvert et assurer un écoulement sanguin adéquat, dans laquelle l'endoprothèse récupérable (100") est configurée pour épouser la forme courbe naturelle du canal artériel et permettre au canal artériel de maintenir sa forme naturelle, l'endoprothèse comprenant :
un connecteur d'extrémité proximale (102") et une pointe d'extrémité distale (106") disposés autour d'un axe longitudinal (A"), le connecteur d'extrémité proximale (102") incluant un ou plusieurs mécanismes de récupération et une lumière de connecteur d'extrémité proximale (104") qui s'étend sur la longueur du connecteur d'extrémité proximale (102"), et la pointe d'extrémité distale (106") présentant une lumière de pointe d'extrémité distale (108") qui s'étend sur la longueur de la pointe d'extrémité distale (106"), et
une pluralité de 4 à 8 entretoises (114a", 114b") s'étendant du connecteur d'extrémité proximale (102") à la pointe d'extrémité distale (106"), chacune des entretoises présentant une extrémité proximale courbe d'entretoise (116") et une extrémité distale courbe d'entretoise (118") dans laquelle l'extrémité proximale courbe (116") et l'extrémité distale courbe d'entretoise (118") s'étendent radialement vers l'extérieur depuis l'axe longitudinal (A") et l'une vers l'autre, chacune des entretoises présentant en outre une portion allongée de corps d'entretoise (120a", 120b") qui s'étend de l'extrémité proximale courbe d'entretoise (116") à l'extrémité distale courbe d'entretoise (118"), les portions allongées de corps d'entretoise (120a", 120b") s'étendant parallèlement les unes aux autres ;
dans laquelle, lorsque l'endoprothèse (100") est positionnée dans le canal artériel, les extrémités proximales courbes d'entretoise (116") et les extrémités distales courbes d'entretoise (118") s'engagent avec le canal artériel pour procurer la force radiale nécessaire pour maintenir le canal artériel ouvert tandis que les portions de corps allongées d'entretoise (120a", 120b") de chacune des 4 à 8 entretoises (114a", 114b") s'engagent minimalement avec le canal artériel pour empêcher l'invagination dans la paroi vasculaire du canal artériel et ainsi permettre une récupération ultérieure, et dans laquelle une ou plusieurs entretoises (114a") présentent une portion de corps allongée (120a") qui se courbe vers l'axe longitudinal (A") et une ou plusieurs entretoises (114b") présentent une portion de corps allongée (120b") qui se courbe à l'écart de l'axe longitudinal (A").

2. Endoprothèse selon la revendication 1, dans laquelle l'extrémité proximale d'entretoise courbe (116") et l'extrémité distale d'entretoise courbe (118") comprennent une courbe sigmoïde.

3. Endoprothèse selon la revendication 1, dans laquelle le mécanisme de récupération comprend une structure de crochet (110") pour recevoir un lasso.

4. Endoprothèse selon la revendication 1, dans laquelle le mécanisme de récupération comprend une moitié d'une attache filetée (112").

5. Endoprothèse selon la revendication 1, comprenant un mécanisme de récupération de crochet (110") et un mécanisme de récupération de moitié d'une attache filetée (112").

6. Endoprothèse selon la revendication 1, dans laquelle la lumière du connecteur d'extrémité proximale (104") et la lumière de la pointe d'extrémité distale (108") présentent chacune un diamètre suffisamment grand pour qu'un fil-guide puisse passer à travers leurs lumières respectives (104", 108").

7. Endoprothèse selon la revendication 1, comprenant 5 entretoises (114").

8. Endoprothèse selon la revendication 1, comprenant 6 entretoises (114").

9. Endoprothèse selon la revendication 1, comprenant une longueur allant de 15 mm à 30 mm.

10. Endoprothèse selon la revendication 1, comprenant une longueur allant de 18 mm à 24 mm.

11. Endoprothèse selon la revendication 1, comprenant un diamètre externe allant de 4 mm à 20 mm.

12. Endoprothèse selon la revendication 1, comprenant un diamètre externe allant de 6 mm à 10 mm.

13. Endoprothèse selon la revendication 1, dans laquelle les entretoises (114") sont formées de nitinol.
